# EUROPEAN PATENT SPECIFICATION

(11) **EP 1 991 292 B1**
(45) Date of publication and mention of the grant of the patent: **04.07.2018**
(21) Application number: 06716374.1
(22) Date of filing: 15.03.2006
(51) Int. Cl.: A61K 35/35, A61M 39/10, A61B 50/31, A61B 50/30

(54) **ADIPOSE TISSUE REGENERATION KIT AND ITS USING METHOD**
FETTGEWEBE-REGENERATIONSSET UND SEIN ANWENDUNGSVERFAHREN
TROUSSE DE RÉGÉNÉRATION DE TISSU ADIPEUX ET PROCÉDÉ D'UTILISATION

(30) Priority: 08.03.2006 KR 20060021839
(43) Date of publication of application: 19.11.2008
(73) Proprietor: Sewon Cellontech Co., Ltd., Youngdeungpo-Gu, Seoul 150-712 (KR)
(72) Inventor: SON, Hyun-Mi, Seoul 137-060 (KR); JANG, Jae-Deog, Seoul 139-784 (KR); CHANG, Cheong-Ho, Seoul 137-040 (KR)
(74) Representative: dompatent von Kreisler Selting Werner - Partnerschaft von Patent- und Rechtsanwälten mbB
(86) International application number: PCT/KR2006/000924
(87) International publication number: WO 2007/102635

(56) References cited:
- WO-A1-03/099412
- WO-A1-2005/046682
- WO-A1-2005/115499
- WO-A2-2005/011569
- FR-A1- 2 853 523
- KR-Y1- 200 323 838
- KR-Y1- 200 333 583
- KR-Y1- 200 368 192
- KR-Y1- 200 408 602
- US-A1- 2003 161 816
- US-A1- 2005 186 193
- US-B1- 6 316 247

## Description

### Technical Field

The present invention relates to a kit for adipose tissue regeneration. More specifically, the present invention relates to a kit for adipose tissue regeneration, which minimizes the risk of infection in fat transplantation for treatment and reconstruction of defect regions using patient's own adipose tissues transplantable into patients and which enables stable surgical operation of adipose tissue transplantation as well as induction of adipose tissue regeneration via constitution of aseptic/sterile implements and a standardized protocol of fat transplantation into a kit in the form of a single set, thereby simultaneously solving the problems suffered by conventional adipose tissue transplantation. Therefore, the present invention accomplishes remarkably improved quality and reliability of the product and thereby is very useful to enhance customer satisfaction.

### Background Art

Document US 2005/186193 A1 discloses a kit according to the preamble of claim 1, comprising a washing container filled with an aseptic and sterile solution and a washing container insertion groove, a first syringe container and a first syringe container insertion groove, a second container and a second syringe container insertion groove, connectors and connecting connector insertion groove, needle and needle container insertion grooves, and a protocol.

According to Melvin A. Shiffman in "Autologous fat transplantation" the first clinical fat transplantation was performed by Neuber. In his medical paper published in 1893, Neuber conducted fat transplantation with some satisfactory results. He collected the almond-sized piece of fat as a filling material, and transplanted it to fill in a depressed area of the face which had resulted from infection of tuberculosis into bones.

Since then, many cases of fat injection under various situations have been attempted by a great number of people. However, all these early attempts led to disappointing results. For example, in 1895, Czemy attempted fat injection to fill a breast defect created by removal of a benign breast tumor. In an article published in 1909, Verderame introduced fat injection into an oculoplastic surgery. However, since most of fat thus injected was absorbed by the body, it was also recommended to inject greater amounts of fat than necessary.

Over about one hundred years since then, a variety of attempts for fat injection have been conducted, but the results exhibited significant variation and difference depending upon surgeons who have published the results of their researches. Therefore, these results have raised disappointment and caused many surgeons to give up, and further, fat injection techniques were confronted with the crisis of being kept secret. However, as numerous methods of reducing body's absorption of fat following fat injection have recently been developed, fat injection techniques have been receiving a great deal of attention again.

Upon scrutinizing various medical articles dealing with fat injection techniques, early attempts and results showed disappointing outcomes with a high absorption rate of more than 50% within a year. Every surgeon who had conducted fat transplantation pointed out a variety of factors that interfere with engrafting of transplanted fat and increase body's absorption of fat. For instance, various suggestions have been continuously made on methods of reducing body's absorption of fat in order to increase an engraft rate of lipoinjection and various methods for enhancing engrafting of fat, including the following various experimental results: differences in an engraft rate depending on from where fat is collected, differences in an engraft rate according to collection methods of fat, experiments to find proper pressure for liposuction, whether washing of collected fat is preferable or not, differences in an engraft rate depending upon injection methods, injection volume or injection mode including a bulk injection or divided injection of fat, and an increased engraft rate by fat injection in combination with addition of betamethasone, insulin or dextran.

In 1928, Hilse has histologically confirmed formation of adipose tissues from free fat grafts without exception and called histocytes filled with fat as lipoblasts. In 1947, Green has treated bone defects secondary to osteomyelitis using fat, and has considered that transplanted fat will become connective tissues and finally undergo ossification, thus being capable of filling in defect regions. In 1956, Peer published his work with autogenous dermal fat grafts. In his work, he has cut a volume of fat corresponding to walnut-sized mass into 20 pieces and transplanted it into defect area. Upon examining the transplanted fat several times for one year, he found that transplantation of bulk fat exhibited a high engraft rate as compared to transplantation of fat in the form of small pieces. To the contrary, Coleman has asserted through his article published in 1995 that when fat is divided into several layers and injected little by little, there are provided more opportunities for supply of oxygen and nutrients and thus it is preferred that fat was portionwise injected in the form of a multilayer.

Further, as mechanical liposuction using cannula has become possible by A. Fisher and G. Fisher in 1974, Bircoll has made an effort to employ the liposuctioned fat in lipoinjection in 1982 and such a method has been being undertaken since then.

Meanwhile, regarding autologous fat transplantation, American Society of Plastic and Reconstruction Surgery (ASPRS) Ad-Hoc Committee on New Procedures (1987) states as follows: autologous fat injection should have historical and scientific basis and is still experimental surgery, and therefore the results of fat injection are too various. As a result, in order to make a conclusion on whether fat injection is useful or not, controlled clinical studies are required.

Conventional autologous fat transplantation, as discussed above, has been still prevalently performed by surgeons in many hospitals and is a transplantation technique for reconstruction of soft tissue defects which is in on-going development. However, throughout the world hitherto, there is no case of combined provision of an easy manipulation method for fat transplantation and aseptic/sterile implements in the form of a single set. Particularly, to the best of our knowledge, no case has been found in the conventional arts wherein a kit for fat transplantation is constituted based on a numerically measurable concept under a standardized protocol. That is, in fact, although current adipose tissue transplantation techniques exhibit considerable advancement over the past adipose tissue transplantation techniques, there is still no significant suggestion of standardized protocols for adipose tissue transplantation or preparatory proceedings for sanitary adipose tissues. Further, there still remain a variety of problems to be solved; that is, currently available adipose tissue transplantation techniques suffer from disadvantages such as necrosis and complications of transplan ted tissues following adipose tissue transplantation, inconvenience and troubles associated with a need for complicated re-surgery due to reduction in the volume of transplanted adipose tissue after surgery, tissue fibrosis, and cell necrosis caused by physical damage.

### Disclosure of Invention

### Technical Problem

Therefore, the present invention has been made in view of the above problems, and it is an object of the present invention to provide a kit for combined provision of an easy manipulation method for fat transplantation and aseptic/sterile implements in the form of a single set concept, which has no precedent throughout the world hitherto, and supply such a kit to domestic and global medical market, thereby securing upgraded technological capability in fat transplantation.

For this purpose, a second object of the present invention is to present a standardized protocol associated with use of a kit for autologous adipose tissue regeneration, which is adapted to enable convenient and easy manipulation thereof.

A third object of the present invention is to minimize problems associated with the risk of infection that may occur upon performing fat transplantation, via aseptic and sterile treatment of all components contained in a kit for adipose tissue transplantation.

A fourth object of the present invention is to induce regeneration of adipose tissues via use of a novel concept of biocompatible material, called Bio-Gel.

A fifth object of the present invention is to present new measures for solving the problems such as necrosis and complications of transplanted tissues, reduction in the volume of transplanted tissues after surgery and the like, via use of biocompatible Bio-Gel to supply nutrients of fat.

Consequently, a sixth object of the present invention is to provide a kit for adipose tissue regeneration, which is suited for enhancing customer satisfaction via remarkably improved quality and reliability of the product.

### Technical Solution

The invention is indicated in the independent claim. A further embodiment is indicated in the dependent claim.

In accordance with an aspect of the present invention, the above and other objects can be accomplished by the provision of a kit for adipose tissue regeneration constituted in the form of a single set kit, wherein the bottom of a kit box equipped with a locking device is provided with a washing container filled with an aseptic and sterile solution for washing adipose tissues and a washing container insertion groove for containing the same; a first syringe container containing first syringes for washing and separation of adipose tissues, injection of the wash solution and mixing of Bio- Gel, respectively, and a first syringe container insertion groove for containing the same; a second syringe container containing a plurality of second Bio-Gel syringes filled with biocompatible materials to promote adipogenesis induction and a second syringe container insertion groove for containing the same; and connector containers containing connectors for use in injection of the wash solution and mixing of washed adipose tissues and biocompatible materials, and connector container insertion grooves for containing the same, and wherein the top of the kit box is provided with needle containers for containing transplantation needles 50 and needle container insertion grooves for containing the same; and a protocol in which standardization of fat transplantation is defined and a protocol insertion groove for containing the same.

The kit for adipose tissue regeneration may be used comprising, filling a total of 4 first syringes (capable of containing 50 mL) having pre-capped inlets for washing and separating of adipose tissues with 25 mL of adipose tissues harvested from donors, respectively; previously filling first syringes (50 mL) for injection of the wash solution arranged in the kit with a solution for washing adipose tissues, injecting 20 mL of an adipose tissue-wash solution into syringes filled with 25 mL of adipose tissues using a syringe connector, and then manually and gently shaking the syringes back and forth 10 to 25 times to wash adipose tissues while capping the inlet; centrifuging the washed adipose tissues so as to be densely concentrated (centrifuging step for centrifuging autologous adipose tissues); after centrifugation, opening the cap of syringe inlet to remove impurities below the adipose tissues, thereby obtaining pure adipose tissues (removing step for removal of impurities); repeating washing, centrifuging and impurity-removing steps; pushing a syringe containing completely impurity-free, densely concentrated adipose tissues and a Bio-Gel arranged in the kit into an adipose tissue syringe using a syringe connector 60, pushing Bio-Gel thereto, drawing a second Bio-Gel syringe, and homogeneously mixing adipose tissues and Bio-Gel by pushing and pulling a first syringe (50 mL) 30 for mixing Bio-Gel, 10 to 20 times, wherein, upon mixing adipose tissues and Bio-Gel, one syringe filled with Bio-Gel is used for mixing per syringe containing 25 mL of adipose tissues (mixing step for mixing autologous adipose tissues and Bio-Gel); and injecting the thus-mixed adipose tissues into a desired target region by a transplantation syringe (transplanting step for transplanting autologous adipose tissues).

### Brief Description of the Drawings

The above and other objects, features and other advantages of the present invention will be more clearly understood from the following detailed description taken in conjunction with the accompanying drawings, in which:
Fig. 1 is a perspective view of a blank kit in which a multitude of various containers in accordance with the present invention were not contained;
Fig. 2 is a perspective view of a kit in which a multitude of various containers applied to the present invention were contained;
Fig. 3 is a cross-sectional view of a first syringe container applied to the present invention;
Fig. 4 is a cross-sectional view of a second syringe container applied to the present invention;
Fig. 5 is an exploded perspective view of a needle container and needle applied to the present invention;
Fig. 6 is an exploded perspective view of a connector container and connector applied to the present invention; and
Fig. 7 is a view showing a state of a connector coupled between a first syringe and a second syringe, applied to the present invention.

### Best Mode for Carrying Out the Invention

The preferred embodiments of the present invention for accomplishing the above- mentioned objects will now be described in more detail with reference to the accompanying drawings.

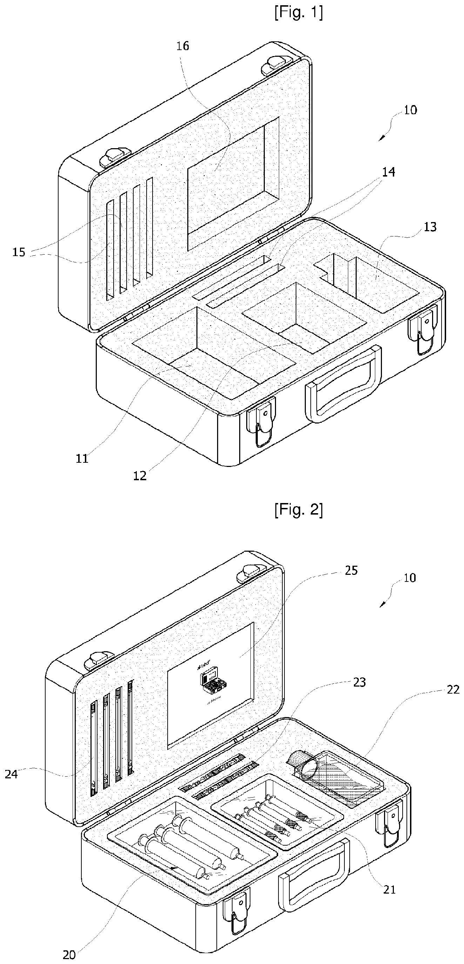

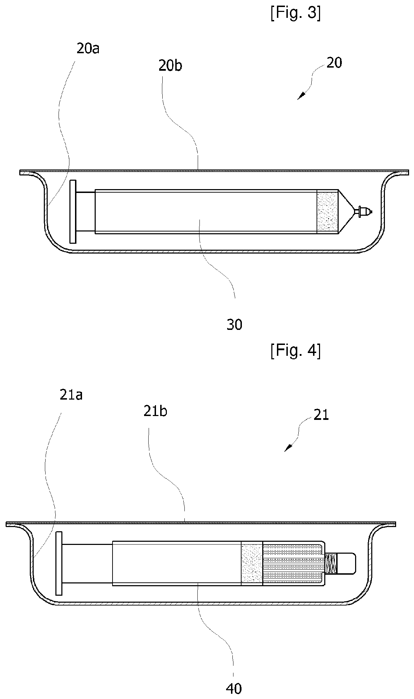

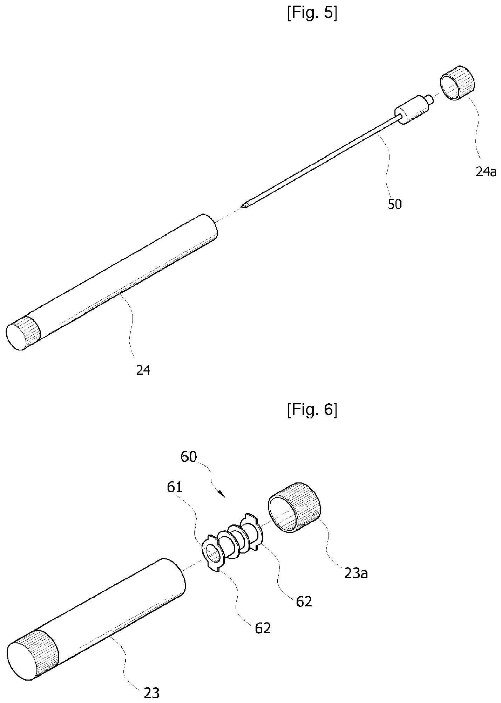

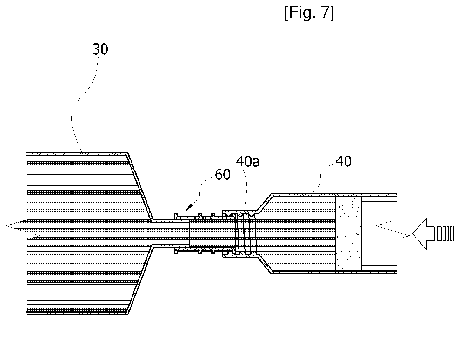

A kit for adipose tissue regeneration, which is applied to the present invention, is constituted as shown in Figs. 1 through 7.

In connection with description of the present invention hereinafter, if it is considered that specific description of known functions or constitution related to the present invention may make the subject matter of the present invention unclear, the detailed description thereof will be omitted.

Terms which will be described hereinafter are established taking into consideration functions in the present invention and may vary according to manufacturer" s intention or general practices in the related art. Therefore, the terms used herein should be defined based on the contents of the specification of the present invention.

First, all of the kits, utilized in the present invention, can reconstruct 100 mL of adipose tissues per kit, and all components are disposable. Components of the kit are products that were sterilized in a space under germ-free conditions, and can be protected against the risk of infection upon performing adipose tissue transplantation.

In addition, in order to completely remove impurities contained in adipose tissues obtained from donors and at the same time, in order to enhance a maximum yield of mature adipocytes and preadipocytes in adipose tissues, there is needed a bottle which is filled with a solution for washing adipose tissues. Here, as the above-wash solution, "a semi-solid gel composition for adipose tissue regeneration" disclosed in Korean Patent Application No. 2005-0090202, which was filed by the present applicant, on September 28, 2005, may be used. This composition is a mixture in which an adipose tissue washed in an adipose tissue-wash solution is in admixture with collagen, an adipogenesis-promoting solution, a serum and a medium.

Further, using the adipose tissue-wash solution of the present invention, it is possible to provide completely impurity-free, pure adipose tissues, which were obtained from donors, and thereby solve the problems associated with adipose tissue necrosis which may occur after adipose tissue transplantation.

In addition, in order to wash adipose tissues, a syringe for injection of the wash solution is previously filled with the adipose tissue-wash solution to achieve convenient washing. In order to wash adipose tissues contained in a syringe for washing and separation of adipose tissues, the wash solution contained in the syringe for injection of the wash solution can be conveniently injected in a specified volume, by using a syringe connector. Further, the syringe connector is used to mix the washed adipose tissues with Bio-Gel constituted in the kit and upon mixing, mixing is handled with a syringe for mixing Bio-Gel.

In order to inject the mixed product of the adipose tissues and Bio-Gel into a target site for fat transplantation, a transplantation needle is necessary.

Further, in order to achieve objects of the present invention, the standardized protocol included in the kit is provided, and procedures of the protocol will be described hereinafter.

The kit of the present invention can be applied to various ranges including humans and animals, and target sites for adipose tissue transplantation may also include a broad area of soft tissue defects such as breast, wrinkles and soft tissue defect regions.

Hereinafter, technical constitution of the present invention will be described in more detail.

In the kit for adipose tissue regeneration according to the present invention, the bottom of a kit box equipped with a locking device is provided with a washing container 22 filled with an aseptic and sterile solution for washing adipose tissues and a washing container insertion groove 13 for containing the same, wherein the solution for washing adipose tissues is filtered and filled into a sterile washing container in an amount of 300 mL and an inlet of the container 22 is sealed again so as to prevent leakage of the wash solution.

In addition, at the bottom of the kit box, a first syringe container 20 containing a plurality of first syringes 30 for washing and separation of adipose tissues, injection of the wash solution and mixing of Bio-Gel, respectively and an first syringe container insertion groove 11 for containing the same are provided. Here, in order to conveniently wash large amounts of adipose tissues, which were obtained from donors, in a lump, and in order to remove impurities from adipose tissues, 50 mL syringes are capped to prevent probable leakage of liquid, sterilized and packaged into a set consisting of 6 syringes.

At the bottom of the kit box is also provided a second syringe container 21 containing a plurality of second Bio-gel syringes 40 filled with biocompatible materials to promote adipogenesis induction and a second syringe container insertion groove 12 for containing the same. 2.5 mL of Bio-Gel for promoting adipogenesis induction and supplying nutrients of fat is filled in a 3 mL syringe which is then sterilized and packaged into a set consisting of 4 syringes.

Further, connector containers 23 containing connectors 60 for use in injection of the wash solution and mixing of washed adipose tissues and biocompatible materials, and connector container insertion grooves 14 for containing the same are provided at the bottom of the kit box. The syringe connector, used for injection of the wash solution into another syringe or for convenient mixing of the washed adipose tissues with biocompatible materials, is sterilized and packaged into a set consisting of 4 or 5 syringe connectors.

Meanwhile, the top of the kit box is provided with a plurality of needle containers 24 containing transplantation needles 50 and a plurality of needle container insertion grooves 15 for containing the same, according to sizes, respectively. In order to transplant the mixed adipose tissues into desired transplantation sites, transplantation needles corresponding to the respective target sites were respectively sterilized and packaged into a set consisting of 3 to 6 needles.

Here, it is preferred to constitute the inside of the first and second syringe containers 20 and 21, needle containers 24 and connector containers 23 in an aseptic and sterile state.

In addition, on the inner circumference of one side of second Bio-Gel syringes 40, a thread part 40a is formed to be engaged with protrusions 62 formed on the outer circumference of the connector 60. The connector 60 is fabricated to have a hole 61 formed at the center thereof, such that the wash solution and the like pass through the hole 61.

Further, at the top of the kit box, a protocol 25 in which standardization of fat transplantation is defined and a protocol insertion groove 16 for containing the same are provided to kit 10 in the form of a single set.

Meanwhile, although the preferred embodiments of the present invention have been disclosed with reference to the accompanying drawings, those skilled in the art will recognize that the present invention may be embodied in different forms with various modifications.

It should be understood that the drawings and detailed description thereof are not intended to limit the invention to the particular form disclosed.

Effects of the a kit for adipose tissue regeneration in accordance with the present invention, as constituted above, will be described hereinafter.

Firstly, by using kit components that can be very easily and conveniently processed according to the standardized protocol and also are sterilized under germ-free conditions, while solving the problems exhibited by conventional adipose tissue transplantation techniques such as a low engraft rate and a body's absorption of transplanted adipose tissues and inducing adipose tissue regeneration, the present invention simultaneously provides solutions to problems of conventional fat transplantation and convenient fat transplantation via use of mixture of processed adipose tissues and adi- pogenesis-promoting Bio-Gel. The respective steps for this purpose will be described prior to introduction of the following Examples.

That is, 25 mL of adipose tissues harvested from donors is filled in a total of 4 first syringes (50 mL) 30 having pre- capped inlets for washing and separating of adipose tissues, respectively.

Thereafter, an adipose tissue- wash solution is previously filled in the first syringe (50 mL) 30 for injection of the wash solution arranged in the kit 10, 20 mL of the adipose tissue- wash solution is injected into a syringe filled with 25 mL of adipose tissues using a syringe connector 60, and then the syringe is gently shaken back and forth with hands 10 to 25 times to wash adipose tissues while capping the inlet of the syringe.

Next, the washed adipose tissues are centrifuged so as to be densely concentrated (centrifuging step for centrifuging autologous adipose tissues). Here, upon centrifuging autologous adipose tissues, it is preferred to perform centrifugation at 1,200 to 3,000 rpm for 5 to 10 min after the washing step.

That is, when centrifugation is carried out at less than 1,200 rpm for less than 5 min, impurities and a fat layer are not distinctively separated to some extent and adipose tissues are not densely concentrated to some extent. Therefore, centrifugation should be carried out at more than 1,200 rpm for more than 5 min. In contrast, when centrifugation is carried out at more than 3000 rpm for more than 10 min, separation between impurities and fat layer is distinctive, but preadipocytes present in adipose tissues may be disrupted. Therefore, it is preferred to perform centrifugation at 1,200 to 3,000 rpm for 5 to 10 min.

After centrifugation, the cap of syringe inlet is open to remove impurities below the adipose tissues, thereby obtaining pure adipose tissues (removing step for removing impurities).

Additionally, washing, centrifuging and impurity removing steps are repeated. These steps are preferably repeated 3 to 4 times.

That is, when these steps are repeated less than 3 times, it is impossible to completely remove blood and other impurities. In contrast, when these steps are repeated more than 4 times, repeated washing and centrifuging may result in tissue necrosis. Therefore, it is preferred to repeat the above washing, centrifuging and impurity-removing steps 3 to 4 times.

Then, a syringe containing completely impurity-free, densely concentrated adipose tissues and a Bio-Gel arranged in the kit are pushed into an adipose tissue syringe using a syringe connector 60, Bio-Gel is pushed thereto, a second Bio-Gel syringe 40 is drawn, a first syringe (50 mL) 30 for mixing Bio-Gel is then pushed and pulled 10 to 20 times to homogeneously mix adipose tissues and Bio-Gel. Here, mixing of adipose tissues and Bio-Gel is carried out in a manner that one syringe filled with Bio-Gel is used for mixing per syringe containing 25 mL of adipose tissues, thereby achieving mixing between autologous adipose tissues and Bio-Gel. As the Bio-Gel, one syringe filled with 2.0 to 3.0 mL of Bio-Gel is preferably used for mixing with adipose tissues.

That is, when the amount of Bio-Gel is less than 2.0 mL, homogeneity of adipose tissue regeneration may be deteriorated due to a low mixing ratio of a Bio-Gel: adipose tissues. Therefore, the amount of Bio-Gel should be more than 2.0 mL. Conversely, when the amount of Bio-Gel is more than 3.0 mL, adipose tissue regeneration may be feasible, but biological properties of regenerated tissues (for example, elasticity) exhibit too high rigidity, which may result in sensation different from original properties intrinsic to gel and tissue fibrosis. Therefore, the amount of Bio-Gel should be less than 3.0 mL.

Finally, the thus-mixed adipose tissues are injected into a desired target region by the corresponding transplantation syringe 50 (transplanting step for transplanting autologous adipose tissues). As discussed above, according to the present invention, it is possible to achieve easier and more convenient adipose tissue transplantation.

### Example

According to the standardized protocol constituted within a kit for adipose tissue regeneration, all relevant procedures need to be performed. For this purpose, 100 mL of abdominal fat liposuctioned from a patient was put into 4 syringes for washing and separating of adipose tissues (25 mL/syringe). When adipose tissues were allocated into syringe, the inlet of the syringe was capped and therefore leakage of adipose tissues from the syringe was prevented upon manipulating the syringe. When adipose tissues were ready to be washed, 50 mL of a solution for washing adipose tissues constituted within the kit was filled in the syringe for injection of the wash solution. The cap of the inlet of the syringe containing adipose tissues to be washed was carefully open and connected to a syringe connector which was sterile packaged. Then, via the syringe connector from the syringe containing the wash solution, 20 mL of solution was transferred to each syringe which was then capped.

Then, the syringes were gently shaken back and forth 10 to 25 times such that the wash solution and adipose tissues were homogeneously mixed to wash adipose tissues. Whether adipose tissues were completely washed was confirmed by naked eyes, followed by centrifuging respective syringes in a centrifuge, at 1,200 to 3,000 rpm for 5 to 10 min. After centrifuging was complete, the cap of syringe inlet was open to remove impurities below the adipose tissue layer. These washing, centrifuging and impurity-removing steps were repeated thrice, thereby obtaining densely concentrated adipose tissues. Then, the thus-obtained densely concentrated adipose tissues were mixed with Bio-Gel which was filled with biocompatible materials inducing adipose tissue regeneration. Mixing with Bio-Gel was carried out by pushing Bio-Gel into the washed adipose tissue syringe, via connection with a syringe connector, replacing the Bio-Gel syringe with a 50 mL syringe for mixing Bio-Gel in the kit, and repeatedly pushing and pulling both syringes 10 to 20 times to thereby effect homogeneous mixing of adipose tissues and Bio-Gel. Upon mixing the washed adipose tissues and Bio-Gel, the syringe connector was disposed and was replaced one by one such that the risk of infection was minimized. Further, in principle, 2.5 mL of Bio-Gel is mixed per 25 mL of adipose tissues. After completion of mixing adipose tissues with Bio-Gel, 4 syringes containing a total volume of 27.5 mL of mixture were prepared and used to reconstruct 100 mL of adipose tissues. After mixing, a size of syringe to be used was selected depending upon transplantation regions, and the mixture for adipose tissue regeneration was directly injected into the desired target region in a divided injection volume.

### Industrial Applicability

As apparent from the above description, the present invention provides a kit for combined provision of an easy manipulation method for fat transplantation and aseptic/sterile implements in the form of a single set concept, which has no precedent throughout the world hitherto, and supply such a kit to domestic and global medical market, thereby securing upgraded technological capability in fat transplantation. In particular, for this purpose, the present invention presents a standardized protocol associated with use of a kit for autologous adipose tissue regeneration, which is adapted to enable convenient and easy manipulation thereof. The present invention enables minimization of problems associated with the risk of infection that may occur upon performing fat transplantation, via aseptic and sterile treatment of all components arranged in a kit for adipose tissue transplantation. Further, the present invention enables induction of regeneration of adipose tissues via use of a novel concept of biocompatible material, called Bio-Gel. In addition, the present invention is to present new measures for solving the problems such as necrosis and complications of transplanted tissues, reduction in the volume of transplanted tissues after surgery and the like, via use of biocompatible Bio-Gel to supply nutrients of fat. Consequently, the present invention provides a kit for adipose tissue regeneration, which is suited for enhancing customer satisfaction via remarkably improved quality and reliability of the product.

## Claims

1. A kit for adipose tissue regeneration constituted in the form of a single set kit (10), wherein the bottom of a kit box equipped with a locking device is provided with: a washing container (22) filled with an aseptic and sterile solution for washing adipose tissues and a washing container insertion groove (13) for containing the same;
a first syringe container (20) containing first syringes (30) for washing and separation of adipose tissues, injection of the wash solution and mixing of a Bio- Gel, respectively, and an first syringe container insertion groove (11) for containing the same;
a second syringe container (21) containing a plurality of second Bio-Gel syringes (40) and a second syringe container insertion groove (12); and connector containers (23) containing connectors (60) for use in injection of the wash solution and mixing of washed adipose tissues and biocompatible materials, and connector container insertion grooves (14) for containing the same, and wherein the top of the kit box is provided with:
needle containers (24) containing transplantation needles (50) and needle container insertion grooves (15) for containing the same; and
a protocol (25) in which standardization of fat transplantation is defined and a protocol insertion groove (16) for containing the same,
**characterized in that**
said second syringe container (21) containing a plurality of second Bio-Gel syringes (40) filled with biocompatible materials to promote adipogenesis induction and a second syringe container insertion groove (12) for containing the same;
wherein the connectors (60) are provided for connecting one first syringe (30) with another first syringe (30) as well as for connecting one first syringe (30) with a second syringe (40), and wherein, on the inner circumference of one side of the second syringe (40), a thread part (40a) is formed to be engaged with protrusions (62) formed on the outer circumference of the connector (60), and the connector has a hole formed at the center thereof, such that the wash solution passes through the hole.

2. The kit according to claim 1, wherein the inside of the first and second syringe containers (20) and (21), needle containers (24) and connector containers (23) is in the aseptic and sterile state.

## Patentansprüche

1. Kit für die Fettgeweberegeneration, das in Form eines Einzelset-Kits (10) vorliegt, wobei der Unterteil eines Kitkastens, der mit einer Verriegelungsvorrichtung ausgestattet ist, versehen ist mit: einem Waschbehälter (22), der mit einer aseptischen und sterilen Lösung gefüllt ist, zum Waschen von Fettgeweben und einer Waschbehälter-Einsetznut (13), die denselben enthalten soll;
einem ersten Spritzenbehälter (20), der erste Spritzen (30) zum Waschen und Abtrennen von Fettgeweben, zur Injektion der Waschlösung bzw. Mischen eines Bio-Gels sowie eine Erster-Spritzenbehälter-Einsetznut (11), die denselben enthalten soll, enthält;
einem zweiten Spritzenbehälter (21), der eine Vielzahl von zweiten Bio-Gel-Spritzen (40) und eine Zweiter-Spritzenbehälter-Einsetznut (12) enthält; und Verbindungsbehältern (23), die Verbindungsstücke (60) enthalten, zur Verwendung bei der Injektion der Waschlösung und zum Mischen der gewaschenen Fettgewebe und biokompatiblen Materialien sowie Verbindungsbehälter-Einsetznuten (14), die dieselben enthalten sollen, und wobei der Oberteil des Kitkastens versehen ist mit:
Nadelbehältern (24), die Transplantationsnadeln (50) enthalten, und Nadelbehälter-Einsetznuten (15), die dieselben enthalten sollen; und
eine Vorschrift (25), in der die Standardisierung der Fetttransplantation definiert ist, sowie eine Vorschrift-Einsetznut (16), die dieselbe enthalten soll;
**dadurch gekennzeichnet, dass**
der zweite Spritzenbehälter (21), der eine Vielzahl von zweiten Bio-Gel-Spritzen (40) enthält, gefüllt mit biokompatiblen Materialien, um die Induktion der Fettbildung zu fördern, und eine Zweiter-Spritzenbehälter-Einsetznut (12), die denselben enthalten soll;
wobei die Verbindungsstücke (60) bereitgestellt sind, um eine erste Spritze (30) mit einer anderen ersten Spritze (30) zu verbinden sowie um eine erste Spritze (30) mit einer zweiten Spritze (40) zu verbinden, und wobei auf dem inneren Umfang einer Seite der zweiten Spritze (40) ein Gewindeteil (40a) so ausgebildet ist, dass es an Vorsprünge (62) angreift, die auf dem äußeren Umfang des Verbindungsstücks (60) gebildet sind, und das Verbindungsstück ein in seiner Mitte gebildetes Loch aufweist, so dass die Waschlösung durch das Loch tritt.

2. Kit gemäß Anspruch 1, wobei sich das Innere des ersten und des zweiten Spritzenbehälters (20) und (21), der Nadelbehälter (24) und der Verbindungsbehälter (23) in einem aseptischen und sterilen Zustand befinden.

## Revendications

1. Trousse pour la régénération de tissu adipeux constituée dans la forme d'une trousse d'ensemble unique (10), dans laquelle la partie inférieure d'une boîte de trousse dotée d'un dispositif de verrouillage est procurée avec : un récipient de lavage (22) rempli d'une solution aseptique et stérile pour laver des tissus adipeux et une rainure d'insertion du récipient de lavage (13) pour le contenir,
un premier récipient de seringue (20) contenant premières seringues (30) pour laver et séparer des tissus adipeux, injection de la solution de lavage et mélanger un Bio-Gel, respectivement, et une rainure d'insertion du premier récipient de seringue (11) pour le contenir,
un deuxième récipient de seringue (21) contenant une pluralité de deuxièmes seringues de Bio-Gel (40) et une rainure d'insertion du deuxième récipient de seringue (12), et des récipients de connexion (23) contenant des connecteurs (60) à utiliser dans l'injection de la solution de lavage et le mélange de tissus adipeux lavés et des matériaux biocompatibles, et des rainures d'insertion des récipients de connexion (14) pour les contenir, et dans laquelle la partie supérieure de la boîte de trousse est procurée de :
récipients d'aiguilles (24) contenant des aiguilles de transplantation (50) et des rainures d'insertion des récipients d'aiguilles (15) pour les contenir, et
un protocole (25) dans lequel la standardisation de la transplantation adipeuse est définie, et une rainure d'insertion du protocole (16) pour le contenir,
**caractérisés en ce que**
ledit deuxième récipient de seringue (21) contenant une pluralité de deuxièmes seringues de Bio-Gel (40) rempli avec des matériaux biocompatibles pour promouvoir l'induction de l'adipogenèse, et une rainure d'insertion du deuxième récipient de seringue (12) pour le contenir,
dans laquelle les connecteurs (60) sont procurés pour connecter une première seringue (30) avec une autre première seringue (30) ainsi que pour connecter une première seringue (30) avec une deuxième seringue (40), et dans laquelle, à la circonférence intérieure d'un côté de la deuxième seringue (40), une partie filetée (40a) est formée pour s'engager contre des saillies (62) formées sur la circonférence extérieure du connecteur (60), et le connecteur présente un trou formé à son milieu, de manière que la solution de lavage passe à travers le trou.

2. Trousse selon la revendication 1, dans laquelle l'intérieur des premier et deuxième récipients de seringue (20) et (21), des récipients d'aiguilles (24) et des récipients de connexion (23) sont dans un état aseptique et stérile.
